Europäisches Patentamt

European Patent Office

Office européen des brevets

(19)

(11) Publication number: **0 144 551**
**A1**

## EUROPEAN PATENT APPLICATION

(21) Application number: **84110382.3**

(22) Date of filing: **31.08.84**

(51) Int. Cl.⁴: **A 61 M 5/28**

(30) Priority: **28.09.83 US 536927**

(43) Date of publication of application:
**19.06.85 Bulletin 85/25**

(84) Designated Contracting States:
**BE DE FR GB IT NL SE**

(71) Applicant: **Becton Dickinson and Company**
**Mack Centre Drive**
**Paramus New Jersey 07652(US)**

(72) Inventor: **Szwarc, Joseph M.**
**71 Grissing Court Cedar Grove**
**Essex County New Jersey(US)**

(74) Representative: **Selting, Günther, Dipl.-Ing. et al,**
**Deichmannhaus am Hauptbahnhof**
**D-5000 Köln 1(DE)**

(54) **Two-component medication syringe assembly.**

(57) A two-component medication syringe (20) with vein indication test capacity includes a barrel (21) having a chamber (44) for retaining fluid and a tip (24) extending from a distal end of the barrel (21) having a passageway therethrough communicating with the chamber (45). The syringe tip (24) is adapted to accept a hypodermic needle. A by-pass stopper (31) is slidably positioned in fluid-tight engagement inside the barrel (21). The barrel (21) also includes a raised peripheral portion (34) serving as a by-pass and defining a by-pass zone. The by-pass zone (34) is longer along the longitudinal axis of the barrel (21) than the length of the by-pass stopper (31) along the longitudinal axis of the barrel (21). The by-pass (34) is positioned so that when the by-pass stopper (31) is within by-pass zone (34), the volume defined within the chamber (45) between the by-pass stopper (31) and the distal end of the barrel (21) is approximately the volume of the combined components of the medication. Further, the by-pass (34) is raised enough to allow fluid to flow around the by-pass stopper (31) when the by-pass stopper (31) is positioned within the by-pass zone (34). Also included is a stopper (36) slidably positioned in fluid-tight engagement inside the barrel (21). This stopper (36) is adapted to engage a plunger rod (37) to facilitate its operation.

FIG.1

EP 0 144 551 A1

# TWO-COMPONENT MEDICATION SYRINGE ASSEMBLY

## BACKGROUND OF THE INVENTION

1.  Field of the Invention.  The present invention relates to a syringe and more particularly concerns a two-component medication syringe with vein indication test capacity.

2.  Description of the Prior Art.  Some injectable medications have rapid loss of potency when they are in their ready-to-use form.  In order to protect against the short shelf life of these medications, many of them are supplied in two-components, and they are mixed at the time of use.  Two-component medication commonly comes in two vials with pierceable stoppers.  A first vial typically contains sterile water and the second vial contains the active ingredients which may be in lyophilized form.  To prepare the medication for use, the user pierces the stopper of the vial containing the water with a sterile syringe and needle assembly and withdraws the water into the syringe.  The needle is then removed from the first vial and inserted into the second vial.  Water is injected into this vial to mix with the lyophilized medication.  The mixed medication is then withdrawn into the syringe for injection.  When the injection is to be made into a vein, it is common practice to insert the needle into the patient and to withdraw the plunger rod slightly from the syringe barrel.  If the needle is in a vein, a slight amount of blood will be drawn into the syringe.  The visual sighting of the blood verifies that the needle is in a vein. This procedure is called the vein indication test. Also, when injection of medication into a vien or artery is not desirable, the vein indication test can be used to assure that the hypodermic needle is not in a vein or artery.

The above recited known components present problems with respect to sterility since only the interior of the medication vial is sterile and bacteria from the exterior of the vials and the environment may be introduced into the medication during the mixing procedure. Also, at the areas where the surface of the lumen of the hypodermic needle intersects the planes of its ground point, there are formed sharp edges that can potentially cut pieces of the rubber vial stopper away as the needle penetrates therein. These pieces of rubber represent a potential health hazard if they pass along with the liquid medication into the patient's body. Further, cost is high since two separate sterile containers and a sterile syringe are normally required.

Brown (U.S. Pat. No. 2,607,344) teaches placing both components of the medication in a glass tube where the components are separated by a stopper with a piston stopper sealing the end of the tube containing a liquid component and a flanged pierceable stopper sealing the end of the tube containing a powder component. Also, within the powder containing compartment, bounded by the stopper and the pierceable stopper, there is a longitudinally positioned groove projecting radially outwardly enlarging the inside diameter of the glass tubing. The groove is longer than the stopper so that when the stopper is positioned within the section of the tube containing the groove, liquid can flow around the stopper through the groove. Also provided is a separate barrel having an open proximal end adapted to accept the glass tube assembly and a distal end with a wall containing a cannula with opposed points. One end of the cannula projects into the barrel, and the other end projects outwardly away from the distal end of the barrel. In use, the tube assembly is inserted into the barrel so that the portion of the cannula inside the barrel penetrates the pierceable stopper establishing fluid communication between the interior of the tube and the atmosphere. Then the piston stopper is forced inwardly pushing the liquid component of the medication and the stopper toward the distal end of the barrel. When the stopper is positioned within the by-pass, the liquid component flows around the stopper through the by-pass to mix with the powder component. To perform the vein indication test, the tube is withdrawn to terminate fluid communication with the cannula and then the outwardly facing portion of the cannula is inserted into the patient. Since the flange of the pierceable stopper is larger than the inside diameter of the barrel, further withdrawal of the tube from the barrel creates a reduced pressure zone between the exterior end of the pierceable stopper and the cannula causing blood to flow from the cannula into the barrel, outside of the tube assembly, if the cannula is lodged in a vein.

-4-

Apparatus and methods for the storage, mixing and administering of two-component medication have been addressed by the prior art, as alluded to, above. However, there is still a need for a simple, straightforward, reliable, easily fabricated syringe assembly, with vein indication test capacity, for the storage, mixing and administering of two-component medications. It is also desirable that the syringe assembly minimize contamination potential by allowing the mixing and administering steps to be performed without puncturing stoppers or transferring the medication components through non-sterile surfaces of the syringe assembly.

## SUMMARY OF THE INVENTION

The two-component medication syringe with vein indication test capacity of the present invention comprises a barrel having a chamber for retaining fluid. A tip extending from the distal end of the barrel has a passageway therethrough for communicating with the chamber and means for accepting a hypodermic needle. A by-pass stopper is slidably positioned in fluid-tight engagement inside the barrel. The barrel also includes by-pass means for allowing fluid to flow around the by-pass stopper when the by-pass stopper is positioned so that the volume defined within the chamber between the by-pass stopper and the distal end of the barrel is approximately the volume of the combined components of the medication. Stopper means is slidably positioned in fluid-tight engagement inside the barrel.

In accordance with another embodiment of the present invention, a two-component medication syringe with vein indication test capacity includes an elongate substantially cylindrical barrel having a chamber for retaining fluid and a tapered tip extending from the distal end of the barrel. This tip has a passageway therethrough communicating with the chamber and means for accepting a hypodermic needle. Sealing means is releaseably connected to the tip for sealing the passageway. A by-pass stopper is slidably positioned in fluid-tight engagement inside the barrel. Also provided is a raised peripheral portion of the barrel serving as a bypass chamber and defining a by-pass zone. Also, the by-pass zone is longer along the longitudinal axis of the barrel than the length of the by-pass stopper along the same axis. The by-pass is positioned so that when the by-pass stopper is within the by-pass zone, the volume defined within the chamber between the by-pass stopper and the distal end of the barrel is approximately the volume of the combined components of the medication. The by-pass is raised enough to allow fluid flow around the by-pass stopper when the by-pass stopper is positioned within the by-pass zone. Also provided is a stopper slidably positioned in fluid-tight engagement inside the barrel and adapted to engage a plunger rod to facilitate its operation. This stopper is capable of moving fluid from the chamber through the passageway upon its movement towards the distal end, and it is further capable of facilitating the drawing of fluid into the chamber through the passageway upon its movement away from the distal end. A first liquid component of medication is contained within the chamber between the by-pass stopper and the stopper. The by-pass stopper is positioned outside of the by-pass zone adjacent to the proximal end of the by-pass. A second component of the medication is substantially within the chamber between the by-pass stopper and the distal end of the barrel.

In accordance with the principles of the present invention, a number of advantages and objectives are attained. Primarily, the present invention provides a simple, straight-forward, reliable, easily fabricated syringe assembly for storing, mixing and administering two component medications. The instant invention minimizes contamination potential by allowing the mixing and administering steps to be performed without puncturing stoppers or transferring the medication components through surfaces which are exterior to the syringe assembly. As will be hereinafter shown, the present invention also has vein indication test capacity.

## BRIEF DESCRIPTION OF THE DRAWINGS

Fig. 1 is a side elevation view of the preferred two-component medication syringe assembly of the present invention;

Fig. 2 is a cross-sectional view of the syringe assembly of Fig. 1 taken along line 2-2;

Fig. 3 is a cross-sectional view of the syringe assembly of Fig. 1 taken along line 3-3;

Fig. 4 is a side elevation view of the syringe assembly, similar to Fig. 2, with tip cap removed and interior section partially exposed, schematically showing the mixing of the two medication components;

Fig. 5 is an enlarged cross-sectional view of the syringe assembly of Fig. 4 similar to the cross-sectional .view of Fig. 3 but with the by-pass stopper in the by-pass zone.

Fig. 6 is a side elevation view of the preferred two-component medication syringe assembly, with interior section partially exposed, showing the position of the stopper and the by-pass stopper when the two components of the medication are fully mixed;

Fig. 7 is a side elevation view of the preferred two-component medication syringe assembly, with hypodermic needle assembly attached and interior section partially exposed, schematically showing the vein indication test;

Fig. 8 is a side elevation view of an alternative embodiment of the preferred two-component medication syringe barrel and by-pass stopper with a radially inwardly projecting by-pass;

Fig. 9 is an enlarged cross-sectional view of the syringe barrel and by-pass stopper of Fig. 8 taken along line 9-9;

Fig. 10 is a side elevation view of another alternative embodiment of the preferred two-component medication syringe barrel and by-pass stopper with a by-pass positioned at an angle with respect to the syringe barrel longitudinal axis;

Fig. 11 is an enlarged cross-sectional view of the syringe barrel and by-pass stopper of Fig. 10 taken along line 11-11;

Fig. 12 is a partial side elevation view of another alternative embodiment of the two-component medication syringe assembly;

Fig. 13 is the two-component medication syringe assembly of Fig. 12 illustrating the shield removed and separated from the syringe barrel; and

Fig. 14 is a cross-sectional view of the two-component medication syringe assembly of Fig. 12 taken along line 14-14.

## DETAILED DESCRIPTION

While this invention is satisfied by embodiments in many different forms, there is shown in the drawings and will herein be described in detail preferred embodiments of the invention with the understanding that the present disclosure is to be considered as exemplary of the principles of the invention and is not intended to limit the invention to the embodiments illustrated. The scope of the invention will be measured by the appended claims and their equivalents.

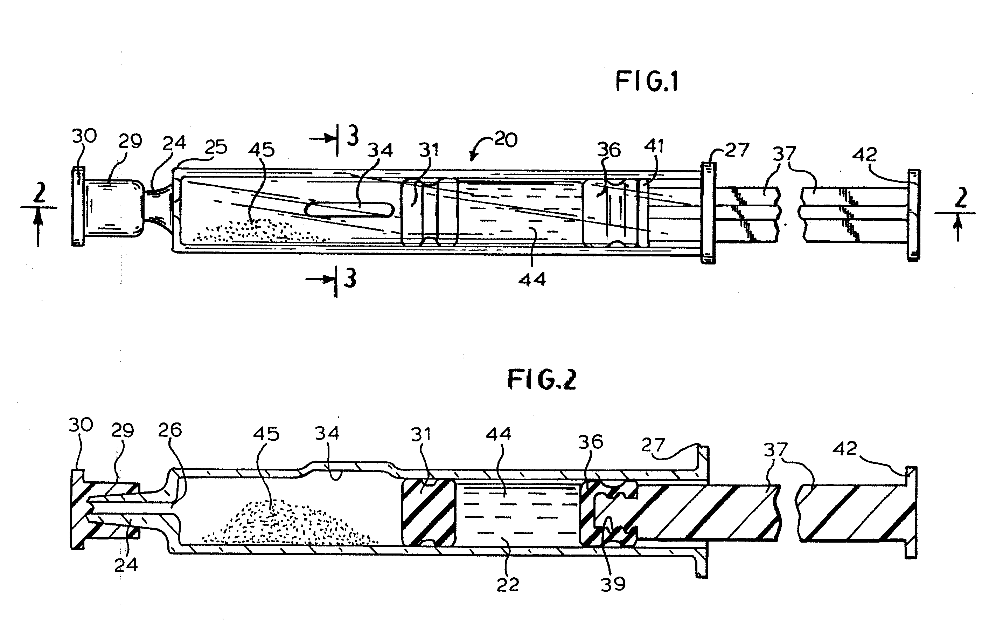

Adverting to Figs. 1 through 3, a two-component medication syringe 20 with vein indication test capacity includes an elongate substantially cylindrical barrel 21 having a chamber 22 for retaining fluid. A tapered tip 24 extends from a distal end 25 of the barrel and contains a passageway 26 therethrough communicating with chamber 22. Tapered tip 24 is adapted to accept a hypodermic needle (not shown). A preferably resiliant tip cap 29 is releasably connected to tapered tip 24 and seals passageway 26 in an air tight manner. Tip cap flange 30 is provided to facilitate installation and removal of the tip cap. A flange 27 is also provided at the proximal end of the barrel to facilitate handling and positioning the syringe.

A flexible by-pass stopper 31 is slidably positioned in fluid tight arrangement inside the barrel. The by-pass stopper outside diameter is larger than the inside diameter of the barrel so that the stopper, when introduced into the syringe barrel, is compressed enough to provide adequate pressure between the syringe and the stopper to seal this interface, but yet remains slidable within the barrel under the influence of force.

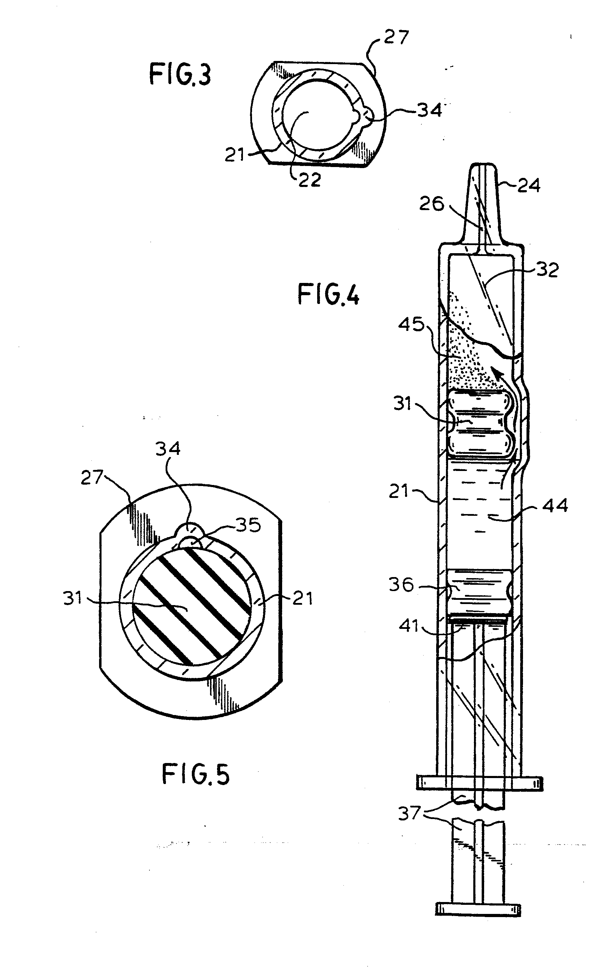

Syringe barrel 21 also includes a by-pass 34 represented by a raised peripheral portion of the barrel extending radially outwardly and which defines by-pass zone Z along the barrel. The by-pass, as best shown in Fig. 3, effectively changes the inside diameter of the syringe barrel as measured through the by-pass zone. Also, the by-pass zone is longer along the longitudinal axis of the barrel than the length of by-pass stopper 31 along the longitudinal axis of the barrel. As will be shown later, the by-pass is large enough to allow fluid flow around the by-pass stopper when the by-pass stopper is positioned within the by-pass zone.

A flexible stopper 36 is slidably positioned in fluid-tight engagement inside the barrel. Stopper 36 is adapted to engage a rigid plunger rod 37. In the preferred embodiment, the stopper contains internal thread 39 which can engage external thread 40 on the plunger rod. The plunger rod is accessible outside of the proximal end of the barrel and is provided to move the stopper along the barrel to force fluid into or out of the chamber through the passageway. Disc-shaped plunger rod flange 42 is provided as a convenient structure for applying forces to move the plunger rod with respect to the syringe barrel. Plunger rod flange 41 is provided to supply a large surface area to transmit force from the plunger rod to the stopper, in a direction toward the stopper, without damaging the stopper. The plunger rod can be installed when the syringe is assembled, or it may be provided as a separate unattached component which is engaged to the stopper at the time of use. It will be apparent to one skilled in the art that numerous constructions can be used to join a stopper and a plunger rod and that the arrangement described above is exemplary of these many possibilities. Also, it is within the purview of this invention to include a one-piece plunger rod-stopper assembly.

The preferred embodiment of the instant invention contains two components of a medication which will be mixed at the time of use. A liquid first component of medication 44 is contained within the proximal end of chamber 22 between by-pass stopper 31 and stopper 36. Note that the by-pass stopper is positioned outside of the by-pass zone adjacent to the proximal end of the by-pass. A second component of medication 45 is contained within the distal end of chamber 22 between by-pass stopper 31 and the distal end of the barrel. The second component of medication may be in the form of liquid, liquid soluble powder or combinations thereof. The preferred embodiment is described with the second component being a lyophilized powder.

It should be noted that minimal force is required to move a flexible stopper along a barrel when it is well lubricated or when the liquid being injected acts as a lubricant. However, when the stopper remains in one position, even for a short period of time, the pressure exerted between the stopper and the syringe barrel tends to force liquid or lubricant out from between the interface between the stopper and the barrel. As a result, the amount of force required to start the stopper moving along the syringe barrel increases dramatically. This increased force is called the breakout force. For many syringes, the breakout force is so high that if the user initially pulls on the plunger rod, it will disengage from the stopper. However, in the instant invention, stopper flange 41 allows the user to provide more force to the stopper, in the direction toward the stopper, than could be applied by the plunger rod in a direction away from the stopper, to facilitate overcoming the breakout force and moving the stopper. It can be seen that the problem with breakout force is compounded where, as with the instant invention, there are two stoppers to be moved.

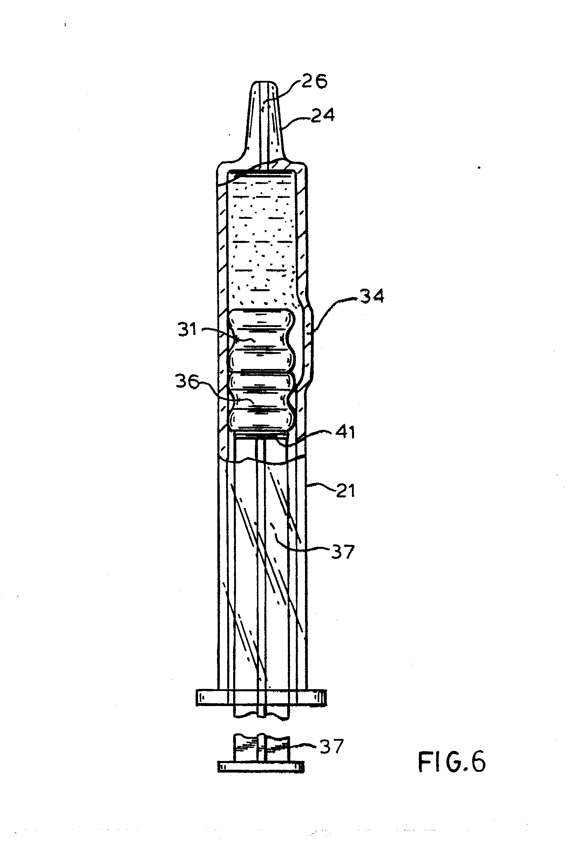

Mixing the first and second components of the medication, as best shown in Figs. 4 through 6, is accomplished by removing the tip cap and orienting the syringe so that the tip faces in an upwardly direction as more specifically illustrated in Fig. 4. The user, while holding barrel 21 in one hand, pushes plunger rod 37 firmly in a direction toward the distal end of the barrel. Once the breakout force of the stoppers is overcome, stopper 36 will move toward the distal end of the syringe barrel, exerting pressure on first component 44, which in turn exerts pressure on by-pass stopper 31. The stopper, first component of medication and the by-pass stopper will continue to move along the barrel until the by-pass stopper is positioned within by the by-pass zone. At this point, the pressure exerted on the liquid first component of the medication by stopper 36 will force the liquid through by-pass passageway 35 between the by-pass and the by-pass stopper, around the by-pass stopper, into the area containing the second component of medication, as best shown in Fig. 4. Motion of the by-pass stopper toward the distal end of the syringe barrel and liquid entering the distal end of the syringe barrel, through the by-pass, will displace any air contained therein and force it out of the barrel via passageway 26. Pressure on the plunger rod is continued until stopper 36 is adjacent to by-pass stopper 31 and all of the liquid component is substantially in distal chamber portion 32 between the distal end of the barrel and the by-pass stopper. At this point, the two components of the medication are mixed, and the medication is ready for injection. As will be pointed out, it is an important feature of the instant invention that the combined volume of the first component and the second component of the medication, when mixed, is approximately equal to the volume defined within distal chamber portion 32 between the by-pass stopper and the distal end of the barrel, when the by-pass stopper is within the by-pass zone. It should be noted that the combined volume of the medication components, when the first component is a liquid and the second component is a solid powder, may be equal to or greater than the volume of the first liquid component depending on the chemical properties of the second powder component.

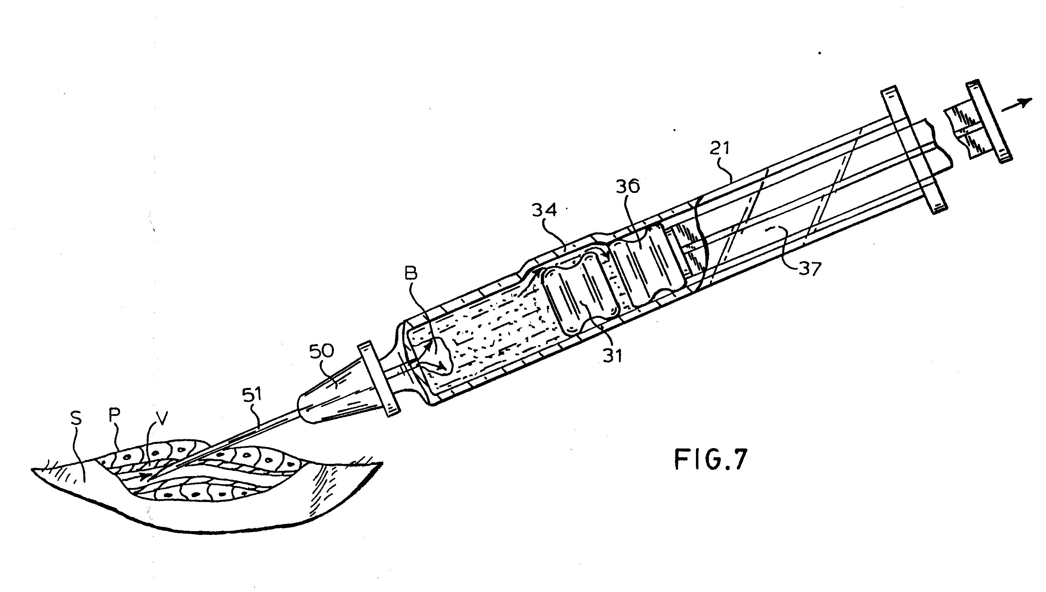

Referring now to Fig. 7, injection of the medication into the vein of patient P requires the placement of a sterile hypodermic needle assembly 50 on the tapered tip of the syringe barrel, and forcing sharp cannula 51 through the patient's skin S into the vein V. To assure that the cannula is properly inserted in the vein, the plunger rod can be drawn away from the distal end of the syringe moving the stopper 36 in that direction to create a reduced pressure zone inside the chamber which will draw blood B from the vein into the chamber. The presence of blood in the chamber will be visual evidence that the cannula is properly placed in a vein. The above described procedure for determining whether or not the cannula is properly placed in a vein is called the vein indication test.

The vein indication test capacity of the instant invention is made possible because the volume of the components of the medication, when mixed, is approximately equal to the volume defined within distal chamber portion 32 between the by-pass stopper and the distal end of the barrel when the by-pass stopper is within the by-pass zone. This volumetric relationship allows the final precise positioning of the by-pass stopper by direct contact with stopper 36 which is controlled by plunger rod 37. Most importantly, when withdrawing the plunger rod to perform the vein indication test, it is only necessary to move stopper 36 and not by-pass stopper 31. Since only one stopper is being moved, the force required to move the plunger rod is less, and there is less chance that the plunger rod will become disengaged from the stopper. Also, when only one stopper is being moved, there is less chance that non-sterile air will leak around stopper 36 due to the lower pressure created within the barrel in attempting to move the stopper along the barrel in a direction toward the proximal end.

The vein indication test should also be performed when injection of medication into a vein or artery is not desirable. In this case, the sharpened cannula of the hypodermic needle, connected to the syringe assembly, is made to pierce the injection site on the patient and the plunger rod is drawn away from the distal end of the syringe, moving stopper 36 in that direction, to create a reduced pressure zone inside the chamber. The absence of blood in the chamber is visual evidence that the cannula is not improperly placed in a vein or artery.

When it is determined that the cannula is properly positioned in the patient, the medication may be injected, in the normal manner, by forcing the plunger rod toward the distal end of the barrel. The motion of plunger rod 37 forces stopper 36 along the barrel, which in turn forces any liquid that may be between stopper 36 and by-pass stopper 31 through the by-pass passageway into the distal chamber portion. At this point stopper 36 is in contact with by-pass stopper 31 and motion of the plunger rod forces both stoppers along the barrel, thus forcing the medication through passageway 26, cannula 51 and into the patient.

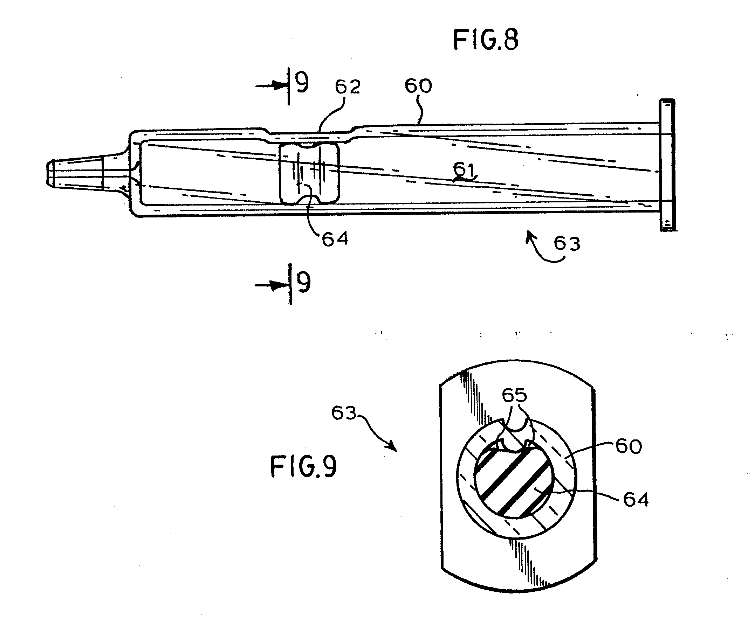

Referring now to Figs. 8 and 9, an alternative syringe 63 of the instant invention includes a syringe barrel 60 having a chamber 61 for retaining fluid and a by-pass 62 represented by a raised peripheral portion of the barrel extending radially inwardly defining a by-pass zone along the barrel. The by-pass zone is longer along the longitudinal axis of the barrel than the length of by-pass stopper 64. The by-pass is large enough to deflect the by-pass stopper so that when the by-pass stopper is in the by-pass zone, liquid can flow around the by-pass stopper through by-pass passageway 65.

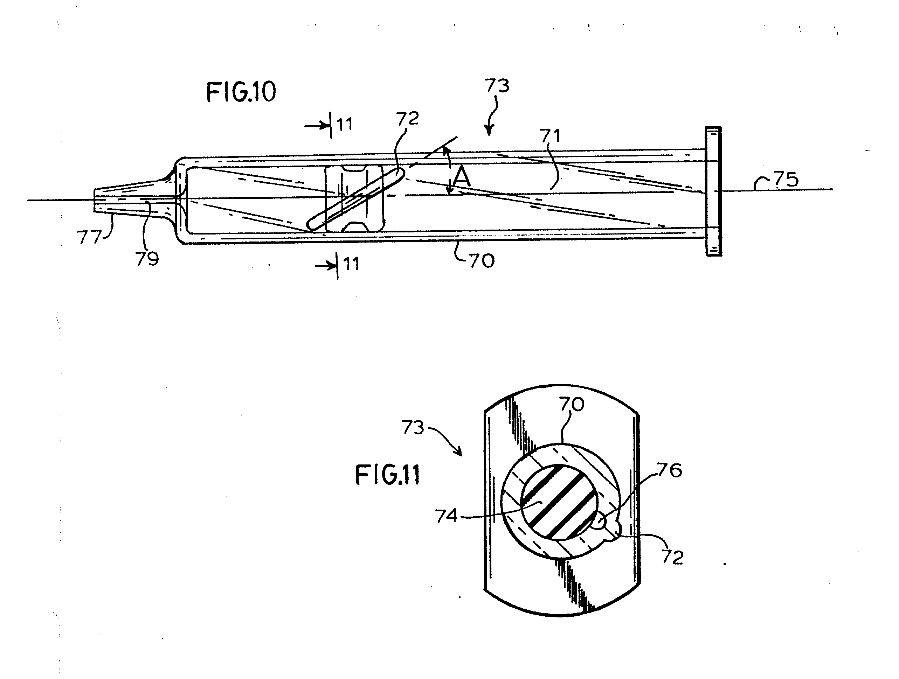

Turning now to Figs. 10-11, another alternative syringe 73 of the present invention includes a syringe barrel 70 having a chamber 71 for retaining fluid and a by-pass 72 represented by a raised peripheral portion of the barrel extending radially outwardly defining a by-pass zone along the barrel. The by-pass zone is longer along longitudinal axis 75 of this barrel than the length of by-pass stopper 74. The by-pass is large enough to allow fluid to flow around the by-pass stopper, through by-pass passageway 76, when the by-pass stopper is positioned in the by-pass zone. The by-pass in this embodiment is positioned at angle A with respect to longitudinal axis 75. The angled by-pass will promote swirling action of the liquid when it enters the portion of the chamber between the by-pass and the distal end of the syringe barrel. The swirling action is caused by the by-pass directing liquid toward the syringe barrel inside surface so that it tends to flow around the inside diameter. This swirling action facilitates the mixing of the two components of the medication. Also, it is not desirable to have liquid medication in passageway 79 of tip 77 before all of the air is expelled from the syringe barrel since this liquid medication can be inadvertently expelled with the air. To this end, the angled by-pass reduces the possibility of liquid entering the passageway, before all air is expelled from the barrell, by directing the liquid medication toward the syringe barrell inside surface.

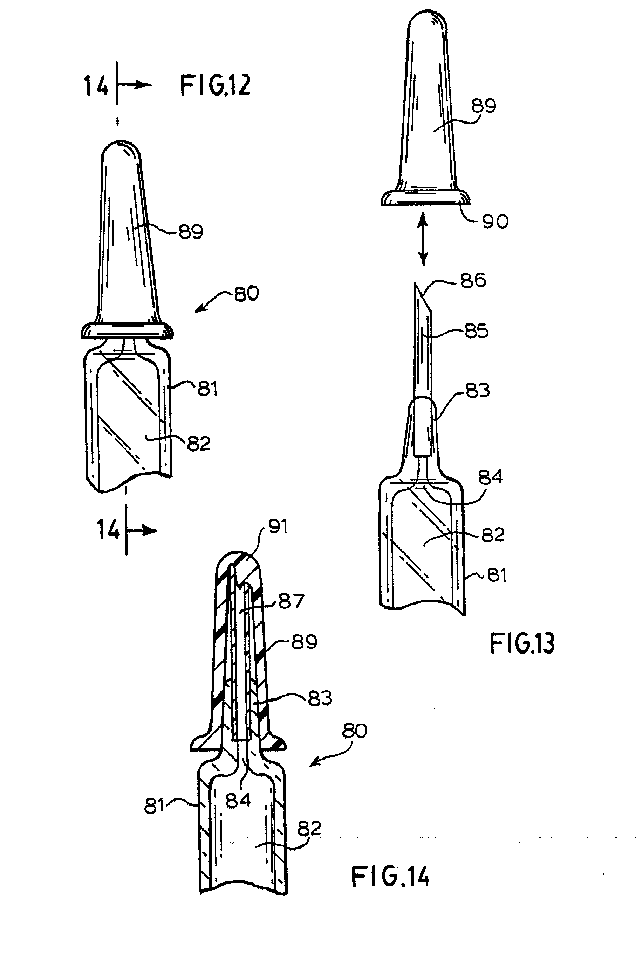

Adverting to Figs. 12-14, another alternative syringe 80 of the present invention includes a syringe barrell 81 having a chamber 82 for retaining fluid and a by-pass (not shown). A tapered tip 83 extends from the distal end of the barrell and contains a passageway 84 therethrough communicating with chamber 82. A needle 85 with a sharp distal end 86 and a lumen 87 is fixedly held in passageway 84, via epoxy, adhesive or other suitable means, so that the lumen is in fluid communication with chamber 82. Flexible needle sheild 89 has an open end 90, a closed end 91 and a receptacle therein. The needle shield is removably attached to tapered tip 83. Shield 89 is sized so that, when it is attached to the tapered tip, sharp disal 86 is embedded in closed end 91 of the needle shield so that lumen 87, and therefore passageway 84, are sealed in an air tight arrangement. Removal of the shield exposes needle 85 and allows flow of fluid from chamber 82 through passageway 84 and lumen 87. The above-described structure eliminates the need for a separate hypodermic needle assembly.

The syringe barrel may be constructed of thermoplastic material such as polypropylene or of glass. The latter material is preferred due to its transparency, low moisture vapor transmission rate and compatability with many medication formulations. A wide variety of materials are suitable for the stopper, by-pass stopper and tip cap with natural rubber and butyl rubber being preferred. The choice of stopper and tip cap material formulations will be highly dependent on compatability with the medication to be stored. A wide variety of rigid materials are suitable for the plunger rod with thermoplastic materials such as polypropylene, polyethylene and polystryrene being preferred. It is preferred that all elements of the two-component medication syringe assembly be sterile when used. Accordingly, materials should be selected for compatability with the sterilization process being used.

Thus, it can be seen that the present invention provides a simple straightforward, reliable, easily fabricated syringe assembly, with vein indication test capacity, for the storage, mixing and administering of two-component medications. The instant invention minimizes contamination potential by allowing the mixing and administering steps to be performed without puncturing stoppers or transferring the medication components through surfaces which are exterior to the syringe assembly.

WHAT IS CLAIMED:

1.    A two-component medication syringe with vein indication test capacity comprising:

a barrel having a chamber for retaining medication;

a tip extending from a distal end of said barrel having a passageway therethrough communicating with said chamber, said tip including means for accepting a hypodermic needle;

a by-pass stopper slidably positioned in fluid-tight engagement inside said barrel; and

a raised peripheral portion of said barrel serving as a by-pass and defining a by-pass zone, said by-pass zone being longer along the longitudinal axis of said barrel than the length of said by-pass stopper along the longitudinal axis of said barrel, said by-pass positioned so that when said by-pass stopper is within said by-pass zone, the volume defined within said chamber between said by-pass stopper and said distal end of said barrel is approximately the volume of the combined components of the medication, said by-pass being raised enough to allow fluid flow around said by-pass stopper when said by-pass stopper is positioned within said by-pass zone.

2.    The two-component medication syringe of Claim 1 further including:

a stopper slidably positioned in fluid-tight engagement inside said barrel adapted to engage a plunger rod to facilitate its operation, said stopper capable of moving fluid from said chamber through said passageway upon its movement toward said distal end, said stopper capable of facilitating the drawing of fluid into said chamber through said passageway upon its movement away from said distal end.

0144551

3. The two-component medication syringe of Claim 1 wherein said chamber has a substantially circularly shaped cross seciton.

4. The two-component medication syringe of Claim 1 wherein said by-pass is raised in an outwardly direction from said barrel.

5. The two-component medication syringe of Claim 1 wherein said by-pass is positioned substantially in alignment with the longitudinal axis of said barrel.

6. The two-component medication syringe of Claim 2 further including sealing means for releasably sealing said passageway.

7. The two-component medication syringe of Claim 6 wherein said sealing means includes a tip cap removably held by said tip.

8. The two-component medication syringe of Claim 6 further including a first medication component in said chamber between said by-pass stopper and said stopper, a second medication component in said chamber between said by-pass stopper and said distal end of said barrel, said by-pass stopper being positioned outside of said by-pass zone adjacent to the proximal end of said by-pass.

9. The two-component medication syringe of Claim 8 wherein said first medication component is liquid.

10. The two-component medication syringe of Claim 9 wherein said second medication component is selected from the group of medications consisting of liquid, powder or combinations thereof.

0144551

11.    The two-component medication syringe of Claim 2
further including a needle having a sharp distal end and a
lumen therethrough, a proximal end of said needle being fixedly
held in said passageway so that said lumen is in fluid communication with said chamber.

12.    The two-component medication syringe of Claim 11
further including a sealing means for releasably sealing
said passageway.

13.    The two-component medication syringe of Claim 12
wherein said sealing means includes a flexible needle shield
having a closed end, an open end and a receptacle therein,
said shield being removably attached to said tapered tip,
said needle being positioned within said receptacle so that
said sharp distal end is embedded in said closed end sealing
said lumen.

14.    The two-component medication syringe of Claim 1
wherein said barrel is made from material selected from the
group consisting of plastic and glass.

15.    The two-component medication syringe of Claim 2
wherein said by-pass stopper and said stopper are made from
material selected from the group consisting of natural rubber
and synthetic rubber.

16.    The two-component medication syringe of Claim 2
further including a rigid plunger rod removably engaged to
said stopper and extending outwardly from the proximal end of
said barrel.

FIG.1

FIG.2

FIG.3

FIG.4

FIG.5

FIG.6

0144551

FIG.7

21 37 36 34 31 B 50 51 V P S

FIG.8

FIG.9

FIG.10

FIG.11

0144551

14 → FIG.12

89

80

81

82

14 →

FIG.13

89

90

86

85

83

84

82

81

91

87

89

83

80

81

84

82

FIG.14

European Patent Office

**EUROPEAN SEARCH REPORT**

| | DOCUMENTS CONSIDERED TO BE RELEVANT | | EP 84110382.3 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | <u>GB - A - 705 392</u> (F.M. TURNBULL) | 1-12, 14-16, | A 61 M 5/28 |
| Y | * Totality * | 13 | |
| Y | <u>CH - A - 502 107</u> (R.W. OGLE) | 13 | |
| | * Fig. 6; column 5, lines 28-32 * | | |
| A | <u>US - A - 2 549 417</u> (F.E. BROWN) | 1-3,5-10,14,16 | |
| | * Totality * | | |
| A | <u>FR - A - 2 076 853</u> (SOCIETE DES USINES) | 1-10, 14-16 | |
| | * Totality * | | |
| | ---- | | **TECHNICAL FIELDS SEARCHED (Int. Cl.4)** |
| | | | A 61 M 5/00 |

The present search report has been drawn up for all claims

| Place of search | Date of completion of the search | Examiner |
|---|---|---|
| VIENNA | 06-03-1985 | LUDWIG |